Europäisches Patentamt

**European Patent Office**

Office européen des brevets

(11) Publication number: **0 125 851**
**A1**

(12) **EUROPEAN PATENT APPLICATION**

(21) Application number: **84303034.7**

(22) Date of filing: **04.05.84**

(51) Int. Cl.³: **D 21 C 9/00**
**A 61 L 15/00**

(30) Priority: **06.05.83 US 492295**

(43) Date of publication of application:
**21.11.84 Bulletin 84/47**

(84) Designated Contracting States:
**BE DE FR GB IT LU NL SE**

(71) Applicant: **PERSONAL PRODUCTS COMPANY**
**Van Liew Avenue**
**Milltown New Jersey 08850(US)**

(72) Inventor: **Brostin, Joel**
**13, Indiana Road**
**Somerset, NJ 08873(US)**

(74) Representative: **Jones, Alan John et al,**
**CARPMAELS & RANSFORD 43 Bloomsbury Square**
**London, WC1A 2RA(GB)**

(54) Absorbent non-delignified wood pulp.

(57) A product for absorbing body fluids and a method for making the same is provided wherein the absorbent body comprises non-delignified wood pulp such as thermomechanical wood pulp. In accordance with the invention, the non-delignified wood pulp is passed through an ionized gaseous medium such as is generated by a corona discharge device whereby the body fluid absorption rate of the non-delignified wood pulp is enhanced.

Absorbent Non-delignified Wood Pulp

Background of the Invention

This invention relates to absorbent products incorporating therein, as a part of the absorbent body of such products, non-delignified wood pulp and more particularly thermomechanical wood pulp.

The use of non-delignified wood pulp such as thermomechnical wood pulp, has already been suggested in products for absorbing body fluids. Several factors make such a material attractive. Firstly, the processes used for converting the raw materials i.e. trees, branches, and the like, into non-delignified wood pulp are substantially more efficient than those for producing delignified wood pulp e.g., chemical wood pulp. It is estimated that in some thermomechanical wood pulp processes up to·90% by weight of the starting material is recovered as pulp as contrasted with recoveries of the order of about 50% for chemical pulping processes. Further, the nonchemical thermomechanical wood pulp processes do not suffer from the problem of creating air and water pollutants or effluents containing such pollutants as is the case of chemical wood pulping processes. This nonpolluting characteristic coupled with the aforementioned increased efficiency, results in a relatively inexpensive form of wood pulp. The low cost then is a major reason for prior suggestions that non-delignified wood pulp be incorporated in such disposable body fluid absorbing products as sanitary napkins, diapers, tampons, wound dressings, and the like.

From a functional view, it has been noted in the art that non-delignified wood pulp fibers are stiffer than chemical

PPC-236

wood pulp and thus have less tendency to collapse and expel absorbed fluid.

The foregoing advantageous features of non-delignified wood pulp notwithstanding, such wood pulp has not yet been employed to any substantial degree in commercial products for absorbing body fluids. The major reason for this is the unfortunate characteristic of such fibers in that the presence of high quantities of lignin surrounding the cellulose fibers render the product substantially hydrophobic as compared to chemical wood pulp. Accordingly, liquid striking the surface of a pad of non-delignified wood pulp tends to either run off or only slowly be absorbed into the body of the pad. The poor wetting characteristics of such wood pulp prevents good capillary liquid transfer within the pad and hence, poor distribution of any of the liquid absorbed. ...

Accordingly, there is a need for providing non-delignified pulp for an absorbent product which not only retains the low cost and good wet collapse resistance of non-delignified pulp but also has improved hydrophilicity.

Summary of the Invention

In accordance with the teachings of this invention, highly absorbent, rapid wicking, non-delignified wood pulp is provided for use in the absorbent body of a product for absorbing body fluids. Such wood pulp is characterized by being exposed to a corona discharge treatment wherein an electric arc is created between charged plates, said arc then ionizing the surrounding gaseous medium. By passing the non-delignified wood pulp through this ionized gaseous medium, the corona, it has been discovered that a substantial increase in the rate of absorption occurs.

-3-

Preferably, thermomechanical wood pulp is employed as the non-delignified wood pulp and, in the simplest form of this invention, the wood pulp is in board form.

It is preferred that the wood pulp surface be subjected to a corona induced by an energy input of at least about 10 kilojoules per square foot of board ($kj/ft^2$) and preferably at least about 50 $kj/ft^2$. Diminishing improvement is noted when the treatment exceeds 250 $kj/ft^2$.

The so treated wood pulp may be incorporated into such body fluid absorbent products as sanitary napkins, tampons, diapers, wound dressings and the like. When the wood pulp is treated in board form it may be used directly in this form or, alternatively, may be ground into wood pulp fluff. Further, the treated material may be combined with other absorbents such as for example, the so-called superabsorbents e.g., hydrocolloidal materials, generally polymers, capable of swelling in aqueous solutions and hence, capable of retaining more than 10 times their own weight of said aqueous solutions. This combination of the treated non-delignified wood pulp and the superabsorbent materials is particularly efficacious in that the rapid wicking property of the treated wood pulp complements the high fluid retention property of the superabsorbents thus promoting rapid fluid uptake with high retentivity and full use of the superabsorbent properties.

Detailed Description of the Invention

The invention comprises providing an absorbent body comprising non-delignified wood pulp that has been treated in the ionized air environment of a corona discharge unit.

-236

The non-delignified wood pulp starting material is generally in the form of thermomechanical wood pulp although other forms of nonchemical wood pulp may be employed e.g. refiner pulp or mechanical wood pulp. The thermomechanical pulping processes have been known for some time and are usually carried out under pressure, using one, two, or more stages of disc refining. Generally such processes employ any of the various soft woods, such as spruce, balsam, western hemlock, douglas fir, white fir and the various pines, especially southern pine such as loblolly and ·slash pines. The wood is initially cut into chips and then undergoes steaming in one or more presteaming stages to soften the middle lamella and allow the wood to be more easily worked and defibrated during the disc refining. The presteamed chips are then pressure-refined at steam temperatures of over 250°F, in one or more stages, to produce the thermal mechanical wood pulp which is generally formed into pulp board. Frequently, such thermomechanical pulp is combined with conventional chemical pulp prior to the board forming operation.

In accordance with the teachings of this invention, the non-delignified wood pulp is subjected to an ionized air environment, preferably generated by corona discharge treatment. It is believed that the operative factor in the improvement in absorption performance achieved through the teachings of this invention is the contacting of the surface of the wood pulp with a sufficient concentration of ionized air and, in particular, oxygen ions, for a sufficient time. When using a corona discharge treatment, the intensity of treatment may be measured by the applied power per unit area per unit time of wood pulp surface being treated or more simply, the applied energy per unit area of surface being treated. This parameter may be expressed, for example, in units of kilojoules per square foot. ($Kj/ft^2$). It should be understood that in using this empirical method of designating the intensity of treatment, the surface area of the wood pulp is defined as the area of a plane passing through the uppermost layer of the wood pulp fibers and not the actual surface area of the fibers themselves. It should also be noted that any other degree of treatment which subjects the wood pulp to substantially the same concentration of ionized oxygen for substantially the same residence time is believed to be an equivalent of the energy per unit area parameters set out herein with respect to corona discharge treatment.

It has been discovered that by treating non-delignified wood pulp in the corona of a corona discharge to the degree represented by an energy input per unit area of at last about 10 $kj/ft^2$ significant improvement is realized in the rate of absorption. Preferably, even more improvement is realized when the energy input is at least about 50 $kj/ft^2$. It has also been found that, beyond an energy input of about 250 $kj/ft^2$, little further improvement is noted.

The invention and the advantages which follows will be better understood by consideration of the following examples.

SAMPLE PREPARATION

Sample 1

A pulp board sheet consisting of 100% thermomechanical wood pulp, obtained from the Eastex Company of Silsbee, Texas is subjected to a corona discharge treatment. The sheet has a basis weight of 1.28 $oz/ft^2$, a width of 8 inches, and a thickness of 0.073 inches. The sheet is introduced, at the rate of one foot per minute into a Lepel High Corona Treatment Unit, said unit being obtained

-6-

from the Lepel High Frequency Laboratory, Inc. of Maspeth, N.Y. and sold by them as Model #ST-2, Type 19110185. Generally, the equipment comprises rollers for feeding the board into the unit, adjustable positive bar electrodes above the sample and a hard conductive rubber drive roller below the sample which acts as the negative electrode. The gap between electrodes may be adjusted by lowering or raising the positive electrode. Means are provided for carrying away the ozone generated by the unit. The sample is treated at a D.C. voltage of 165V a D.C. amperage of 1.2 to 2 amp., with the gap set at 3/16 inches. Several samples of the Eastex TMP board are treated at various energy input levels of from 0 to about 475 $kj/ft^2$.

Sample 2

A pulp board sheet consisting of 50% thermomechanical wood pulp and 50% bleached chemical wood pulp, obtained from the Weyerhauser Company of Seattle, Washington, is subjected to a corona discharge treatment. The sheet has a basis weight of 1.98 $oz/ft^2$ a width of 5.5 inches, and a thickness of 0.044 inches. The sheet is introduced into the above described Lepel High Corona Treatment Unit, again at a rate of one foot per minute. The sample is treated at a D.C. voltage of 165V a D.C. amperage of 3.8 amp, with a gap set at 3/16 inches. Several samples of the 50-50 board are treated at various energy input levels of from 0 to 950 $kj/ft^2$.

Sample 3 (Comparative)

As a comparative sample, a pulp board sheet consisting wholly of chemical wood pulp and specifically, 100% bleached kraft wood pulp obtained from the ITT Rayonier Company of Stanford, Connecticut, is subjected to a corona discharge treatment. The sheet has a basis weight of

2.24 oz/ft$^2$, a width of 5.5 inches, and a thickness of 0.051 inches.  The sheet is introduced into the Lepel High Corona Treatment Unit at a rate of one foot per minute and is treated at a D.C. voltage of 165V, a D.C. amperage of 1.2-2, amp, with a gap set at 3/16 inches.  Several samples of this wholly chemical wood pulp board are treated at various energy input levels of from 0 to about 950 kj/ft$^2$.

Example 1

The sample 1 series of pulp board sheets consisting of corona treated 100% thermomechanical wood pulp are tested for their relative absorption rates utilizing the 7 cc Absorption Test.  In accordance with this testing procedure, the sample is placed on an absorbent substrate such as a chemical wood pulp pad.  A half inch thick Plexiglas plate having an eliptical orifice therethrough is placed upon the sample to be tested.  The eliptical orifice has a major axis of 1 1/2 inches and a minor axis of 3/4 inches.  An aqueous solution having a salt content of 1.0 percent sodium chloride, by weight, is poured into the orifice while keeping the orifice filled until a total of 7 ccs of liquid has been applied.  Using a stopwatch, the time from the initial contact of liquid with the sample to when the last of the fluid is absorbed from the orifice is measured and recorded as the absorption time. Each sample tested measured 5 centimeters by 22.5 centimeters.  Table 1 below reports the absorption time as a function the intensity of the corona discharge treatment expressed as kj/ft$^2$.

-8-

## Table 1

### Absorption Rate, 7 cc Absorption Test
### on Sample 1, 100% Thermomechanical Pulp

| Intensity of Treatment $(kj/ft^2)$ | Absorption Time (sec.) |
|---|---|
| 0 | 10.4 |
| 23.8 | 9.3 |
| 47.5 | 5.6 |
| 190.0 | 5.0 |

As Table 1 clearly shows, the increase in intensity of treatment results in a substantial decrease in the absorption time until a point where further treatment has essentially no additional effect.

### Example 2

The Sample 1 boards comprising corona discharge treated, 100% thermomechanical wood pulp, are tested for their wicking rate using the 15° Wicking Plate Test. In accordance with this test, a strip of board measuring 1.5 by 10 centimeters is placed on a plate forming an angle of 15° with the horizontal. The plate rests on a horizontal plate having an orifice therein, the orifice being in flow communication with a reservoir of aqueous solution having a salt concentration of 1.0% sodium chloride, by weight. The solution level is maintained level with the surface of the plate. The edge of the sample nearest the plate is adjusted to touch the surface of the fluid in the orifice. The time required for the fluid to wick upward through the board is measured for each centimeter that the fluid front traverses. The results of this test are reported below as

PPC-236

the average wicking rate in centimeters per minute as a function of the intensity of corona discharge treatment.

Table 2

Wicking Rate, 15$^{a}$ Wicking Plate Test
on Sample 1, 100% Thermomechanical Pulp

| Intensity of Treatment (kj/ft$^2$) | Wicking Rate (cm./min.) |
|---|---|
| 0 | 4.8 |
| 23.8 | 7.0 |
| 47.5 | 7.8 |
| 190.0 | 11.1 |

As Table 2 indicates, increasing intensity in treatment increases the rapidity of wicking.

Example 3

Each of the series of samples 1-3, i.e., the 100% thermomechanical wood pulp, the 50% thermomechanical and 50% chemical wood pulp, and the 100% chemical wood pulp samples are tested for their absorbency rates using the Basket Sink Test. In accordance with this test, a cylindrical wire basket is filled with a 5 gram sample of disintegrated test material, i.e., pulp board which has been fluffed in a Waring blender. The basket is held above a reservoir of water with the axis of the basket parallel to the surface of the water. The basket is then dropped into the water, and the time required for the basket to be fully submerged is reported as the basket sink time. Samples are tested immediately after corona discharge treatment and then after 1 week, 3 weeks, and 5 weeks after such treatment. The results are reported

PPC-236

below in Table 3 as a function of the intensity of treatment.

## Table 3

### Absorbency Rate by Basket Sink Test
### Effect of Aging

| Sample | Intensity $(kj/ft^2)$ | Basket Sink Time (Sec.) | | | |
|---|---|---|---|---|---|
| | | Unaged | 1 Week | 3 Weeks | 5 Weeks |
| Sample 1 | 0 | 62 | 74 | 81 | 118 |
| (100% | 47.5 | 56 | 59 | 77 | 105 |
| Thermomechanical) | 95.0 | 47 | 46 | 73 | 104 |
| Pulp | 143 | 48 | 40 | 62 | 103 |
| | 190 | 49 | 43 | 54 | 94 |
| | 238 | 40 | 42 | 43 | 91 |
| | 475 | 27 | 32 | 36 | 73 |
| Sample 2 | 0 | 4.7 | 6.6 | 7.3 | 8.0 |
| (50% Chemical, | 47.5 | 3.4 | 6.1 | 6.0 | 6.7 |
| 50% Thermomechanical) | 95.0 | 3.3 | 4.3 | 4.8 | 5.5 |
| Pulp | 190.0 | 3.0 | 3.6 | 4.1 | 4.3 |
| | 285.0 | 2.8 | 3.4 | 3.6 | 3.9 |
| | 380.0 | 2.5 | 3.3 | 3.4 | 3.6 |
| | 475.0 | 2.4 | 3.0 | 3.2 | 3.4 |
| | 950.0 | 2.3 | 2.8 | 3.0 | 3.3 |
| Sample 3 | 0 | 1.4 | 1.5 | 1.5 | 1.5 |
| (100% Chemical) | 47.5 | 1.1 | 1.5 | 1.5 | 1.5 |
| Pulp | 475.0 | 1.0 | 1.5 | 1.5 | 1.5 |
| | 950.0 | 1.0 | 1.5 | 1.5 | 1.5 |

As Table 3 indicates, Sample 1, the 100% thermomechanical wood pulp exhibits a substantial decrease in sink time, i.e., an increase in absorbency rate as the intensity is increased. This relationship holds true even after aging for periods of 1-5 weeks indicating a permanent improvement in the absorbency rate by virtue of the corona discharge treatment.

Sample 2, comprising 50% thermomechanical wood pulp and 50% chemical wood pulp, will exhibit better absorbency rates by virtue of the presence of the hydrophilic chemical wood pulp. Nevertheless, substantial improvement is seen in the absorbency rate both initially and after being aged by increasing the intensity of treatment. It should be noted that this improvement diminishes at the higher intensity rates.

Sample 3, the comparative sample comprising of 100% chemical wood pulp, likewise shows an improvement in absorption rate immediately after treatment. It is noted, however, that after a period of 1 to 5 weeks, this initial improvement has disappeared and there is no difference in absorption rate irrespective of the degree of treatment.

CLAIMS

1. An absorbent body, for use in a product for absorbing body fluid, comprising non-delignified wood pulp, said wood pulp having been treated by passing said wood pulp through an ionized gaseous medium whereby the body fluid absorption rate of said wood pulp is increased.

2. The body of claim 1 wherein said wood pulp is passed through the corona of a corona discharge apparatus.

3. The body of claim 2 wherein said wood pulp is treated in board form.

4. The body of claim 3 wherein said board is treated at an intensity equivalent to an energy input to the corona discharge unit of at least about 10 kj/ft$^2$ of board surface.

5. The body of claim 4 wherein said board is treated at an intensity equivalent to an energy input to the corona discharge unit of at least about 50 kj/ft$^2$ of board surface.

6. The body of claim 5 wherein said board is treated at an intensity equivalent to an energy input ranging from about 50 to about 250 kj/ft$^2$ of board surface.

7. The body of any one of claims 1 to 6 wherein said non-delignified wood pulp is thermomechanical wood pulp.

8. The body of any one of claims 1 to 6 wherein said non-delignified wood pulp is mechanical wood pulp.

9. The body of any one of claims 1 to 6 wherein said non-delignified wood pulp is refiner wood pulp.

10. The body of any one of claims 1 to 9 wherein said absorbent body comprises superabsorbent material.

11. A method of manufacturing an absorbent body comprising:

subjecting non-delignified wood pulp to an ionized gaseous medium to substantially increase the body fluid absorption rate of said wood pulp; and

forming an absorbent body comprising said treated non-delignified wood pulp.

12. The method of claim 11 wherein said non-delignified wood pulp is treated in board form.

13. The method of claim 12 wherein said non-delignified wood pulp is subjected to an intensity equivalent to the ionized gas corona of a corona discharge unit induced by an energy input of at least 10 kj/ft$^2$ of said board.

14. The method of claim 13 wherein said energy input is at least 50 kj/ft$^2$ of said board.

15. The method of claim 13 wherein said energy input ranges from about 50 to about 250 kj/ft$^2$ of said board.

16. The method of any one of claims 11 to 15 wherein said non-delignified wood pulp in thermomechanical wood pulp.

17. The method of any one of claims 11 to 15 wherein said non-delignified wood pulp is mechanical wood pulp.

18.     The method of any one of claims 11 to 15 wherein said non-delignified wood pulp is refiner wood pulp.

19.     The method of any one of claims 11 to 18 wherein superabsorbent material is incorporated into said absorbent body.

20.     A product for absorbing body fluids, comprising an absorbent body according to any one of claims 1 to 10.

**0125851**

Application number

**European Patent Office**

**EUROPEAN SEARCH REPORT**

| | DOCUMENTS CONSIDERED TO BE RELEVANT | | EP 84303034.7 |

| Category | Citation of document with indication, where appropriate, of relevant passages | Relevant to claim | CLASSIFICATION OF THE APPLICATION (Int. Cl. ³) |
|---|---|---|---|
| X | AU - B - 454 322 (COMMONWEALTH RES. ORG.) <br> * Claims 4-6,14; page 2, lines 5-10 * | 1,2,11, 20 | D 21 C 9/00 <br> A 61 L 15/00 |
| A | * Claims 1,4 * | 3-9,12, 18 | |
| A | US - A - 3 806 404 (LIEBERGOTT) <br> * Abstract; examples 2(b),2(c) * | 1,11, 17 | |
| A | US - A - 3 554 825 (GORING) <br> * Claims 1,7; column 3, lines 7-9 * | 1,11 | |
| A | GB - A - 1 096 836 (THIELEN) <br> * Claim 1 * | 10,19 | TECHNICAL FIELDS SEARCHED (Int. Cl. ³) |
| A | US - A - 3 817 701 (THORSEN) | | D 21 C <br> A 61 L <br> D 21 H <br> D 06 M <br> A 61 F |
| A | DE - A1 - 2 929 512 (KENOGARD) | | |

The present search report has been drawn up for all claims

| Place of search | Date of completion of the search | Examiner |
|---|---|---|
| VIENNA | 30-07-1984 | HOCHHAUSER |